# EUROPEAN PATENT SPECIFICATION

(11) **EP 1 755 612 B1**
(45) Date of publication and mention of the grant of the patent: **21.08.2013**
(21) Application number: 05744802.9
(22) Date of filing: 13.05.2005
(51) Int. Cl.: A61K 31/551, A61K 31/5517, A61P 35/02

(54) **Pyrrolobenzodiazepine therapeutic agents useful in the treatment of leukaemias**
Therapeutische Pyrrolobenzodiazepine zur Behandlung von Leukämie
Agents thérapeutiques de pyrrolobenzodiazépine utiles dans le traitement des leucémies

(30) Priority: 13.05.2004 GB 0410725
(43) Date of publication of application: 28.02.2007
(73) Proprietor: Spirogen Sàrl, St-Légier-La Chiésaz (CH)
(72) Inventor: PEPPER, Christopher John, Vale of Glamorgan CF64 5QD (GB); THURSTON, David Edwin, London Greater London WC1N 1AX (GB)
(74) Representative: Watson, Robert James
(86) International application number: PCT/GB2005/001881
(87) International publication number: WO 2005/110423

(56) References cited:
- EP-A- 1 193 270
- WO-A-93/18045
- WO-A-2005/040170
- WO-A2-2005/105113
- PEPPER CHRISTOPHER J ET AL: "The novel sequence-specific DNA cross-linking agent SJG-136 (NSC 694501) has potent and selective in vitro cytotoxicity in human B-cell chronic lymphocytic leukemia cells with evidence of a p53-independent mechanism of cell kill" CANCER RESEARCH, vol. 64, no. 18, 15 September 2004 (2004-09-15), pages 6750-6755, XP008055772 ISSN: 0008-5472
- ALLEY MICHAEL C ET AL: "SJG-136 (NSC 694501), a novel rationally designed DNA minor groove interstrand cross-linking agent with potent and broad spectrum antitumor activity. Part 2: Efficacy evaluations" CANCER RESEARCH, vol. 64, no. 18, 15 September 2004 (2004-09-15), pages 6700-6706, XP008055764 ISSN: 0008-5472
- GREGSON S J ET AL: "Design, Synthesis, and Evaluation of a Novel Pyrrolobenzodiazepine DNA-Interactive Agent with Highly Efficient Cross-Linking Ability and Potent Cytotoxicity" JOURNAL OF MEDICINAL CHEMISTRY, AMERICAN CHEMICAL SOCIETY. WASHINGTON, US, vol. 44, no. 5, 2001, pages 737-748, XP002272009 ISSN: 0022-2623
- GREGSON S J ET AL: "Linker Length Modulates DNA Cross-Linking Reactivity and Cytotoxic Potency of C8/C8' Ether-Linked C2-exo-Unsaturated Pyrrolo[2,1-c][1,4]benzodiazepine (PBD) Dimers" JOURNAL OF MEDICINAL CHEMISTRY, AMERICAN CHEMICAL SOCIETY. WASHINGTON, US, vol. 47, February 2004 (2004-02), pages 1161-1174, XP002316295 ISSN: 0022-2623
- SMELLIE M ET AL: "CELLULAR PHARMACOLOGY OF NOVEL C8-LINKED ANTHRAMYCIN-BASED SEQUENCE-SELECTIVE DNA MINOR GROOVE CROSS-LINKING AGENTS" BRITISH JOURNAL OF CANCER, LONDON, GB, vol. 70, 1994, pages 48-53, XP009033679 ISSN: 0007-0920

## Description

The present invention relates to a pyrrolobenzodiazepine (PBD) dimer therapeutic agent useful in the treatment of leukaemias, especially B-cell leukaemias, that exhibit a resistance to other chemotherapeutic drugs.

### Background

The development of drug resistance is one of the most important problems encountered in cancer chemotherapy as up to 50% of patients cancers have de novo drug resistance or develop resistance to anticancer drugs.

Studies indicate that most, if not all, chemotherapeutic agents exert their anticancer activity by inducing apoptosis; therefore resistance to apoptosis may be a major factor limiting the effectiveness of anticancer therapy (Curr. Med. Chem. Anti-Canc. Agents, 2002, 2(3), 387-401). Much research has been conducted into apoptosis as an active mechanism of cell death (Wien. Klin. Wochenschr., 2003, 115(15-16), 563-574) and into the clinical use of DNA damaging agents to activate tumour suppression responses triggered by DNA damage to induce apoptosis in damaged cells (Apoptosis, 2000, 5(6), 491-507).

Resistance to the cytotoxic effects of chemotherapeutic agents has been observed in a number of different human cancers. Drug resistance has manifested itself in human leukaemias as both single drug resistance (Oncogene, 2003, 22(47), 7389-7395; Curr. Opin. Hematol., 2002, 9(4), 303-307; Leukaemia, 2003, 17(9), 1794-1805) and multidrug resistance (Int. J. Hematol., 2000, 72(3), 290-297). Many different mechanisms have been proposed for how cancers, and especially liquid malignancies, develop drug resistance (Vet. Clin. North Am. Small Anim. Pract., 2003, 33(3), 651-667).

Tumour suppression responses are thought to be regulated, at least in part, by the p53 protein. This may act either to initiate DNA repair mechanisms or to activate mechanisms that lead to apoptotic destruction of the cell (Cancer Lett., 1998, 131(1), 85-99). However some tumours are p53 mutant or p53 null hence reducing or even eliminating the p53 mediated pathway as a possible mechanism for control of apoptosis.

It is known that some of the chemotherapeutics used in the treatment of B-cell leukaemias deplete immunological T-cells as well as killing malignant B-cells. This has been shown for fludarabine (Blood, 1998, 91(5), 1742-1748; Br. J. Hematol., 1995, 91(2), 341-344) and for chlorambucil (Cell Cycle, 2003, 2(1), 53-58).

Many chemotherapeutics also show significant cytotoxicity towards non-malignant cells as well as inducing apoptosis in malignant cells.

Pyrrolobenzodiazepines (PBDs) are known in the art, some of which have the ability to recognise and bond to specific sequences of DNA. PBDs are of the general structure:

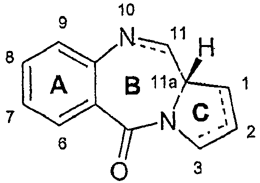

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (*S*)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

Gregson et al. in J. Med. Chem. (2001) and J. Med. Chem. (2004)describe pyrrolobenzodiazepine dimers having cytotoxic effect against a number of leukaemic cell lines.

EP 1193270 discloses the use of SJG-136 for treating cisplatin-resistant cancer such as leukaemia.

WO 2005/105113 describes the use of a pyrrolobenzodiazepine derivative combined with fludarabine for the treatment of cancer, especially leukaemia.

### Summary of the invention

The present invention provides a cytotoxic agent that induces apoptosis preferentially in malignant cells over non-malignant cells in a mixture of malignant and non-malignant cells either in vitro or in vivo from a patient with B-cell chronic lymphocytic leukaemia.

The compound of the invention is of formula I:

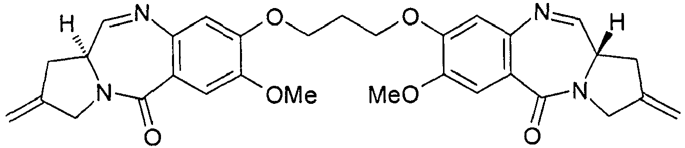

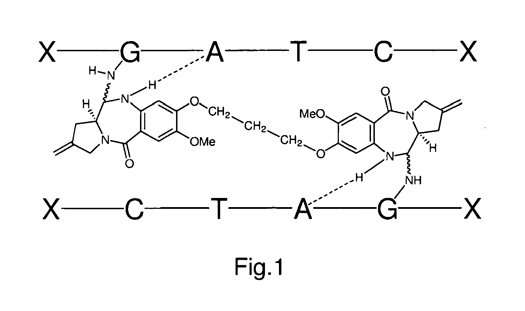

The compound formula I is known to interact in the minor groove of DNA to form a cross link between bases located on opposite strands of the DNA. It is believed that the molecular structure of the compound of formula I allows hydrogen bonding interactions between the compound and certain molecular features of the DNA bases as shown in fig. 1

The present invention relates to a compound of formula I or pharmaceutically acceptable salt or solvate thereof for use in a method of treating B-cell ,wherein it is desired to selectively kill malignant B-cells.

An important aim in terms of drug development for the treatment of cancer is to produce a compound that selectively kills cancer cells without inducing significant cytotoxicity in the surrounding normal tissue. Unfortunately, no differential tumour-selective therapeutic index exists for most of the currently used drugs and substantial normal tissue damage is encountered during cancer therapy. This can be one of the most significant limiting factors in determining the dose and schedule of a new agent. Naturally, individual cell types have different susceptibilities to anticancer drugs and this is dependant on a number of factors including the proliferative index of the cells and their innate tendency to succumb to or resist cell death signals.

The compound and pharmaceutically acceptable compositions of the present invention show a higher cytotoxicity towards malignant B-CLL cells than towards normal B-cells. Preferably the ratio of the LD₅₀ for the compound and pharmaceutically acceptable compositions of the present invention in B-CLL cells and the LD₅₀ in normal B-cells is at least 2:1, preferably at least 5:1, more preferably at least 10:1.

The present invention also relates to the use of the PBD dimer of formula I in the manufacture of a medicament for the treatment of B-cell chronic lymphocytic leukaemia wherein it is desired to selectively kill malignant B-cells.

In other words, the compound of formula I is useful in the manufacture of a chemotherapeutic agent for the treatment of B-cell chronic lymphocytic leukaemia wherein it is desired to selectively kill malignant B-cells.

### Description of the figures

Figure 1 illustrates a possible binding mode for the compound of formula I to DNA.

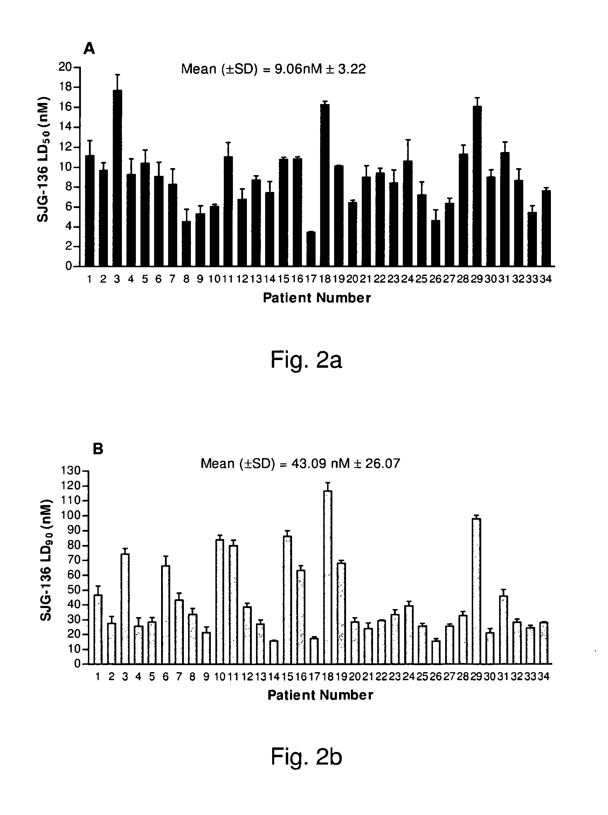

Figure 2 shows a comparison of the in vitro sensitivity to a compound of formula I (SJG-136) of samples taken from 34 B-CLL patients. Figure 2a shows cytotoxicity compared using LD₅₀ values (± SD). Figure 2b shows LD₉₀ values (± SD) derived from in vitro cultures of B-CLL cells exposed to SJG-136 (10⁻¹⁰-10⁻⁷ M) for 48h. Results represent the means (± SD) of three independent experiments.

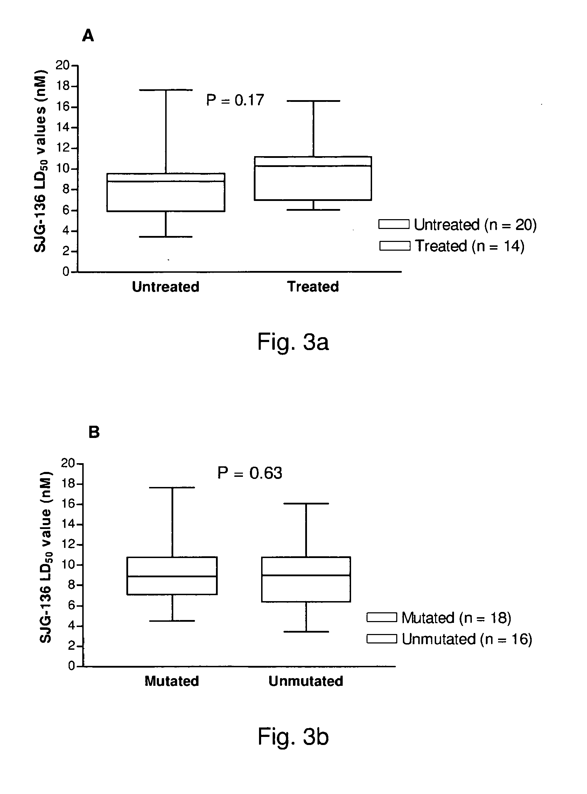

Figure 3 illustrates a comparison of the cytotoxic effect of a compound of formula I (SJG-136) on treated versus untreated and V_{H} gene mutated versus unmutated B-CLL cells. Figure 3a shows the mean LD₅₀ values calculated from the dose-response curves derived from a flow cytometric apoptosis assay indicating drug sensitivity between previously treated and untreated B-CLL cells. Figure 3b shows the cytotoxicity of SJG-136 in B-CLL samples derived from patients with mutated or unmutated immunoglobulin V_{H} genes.

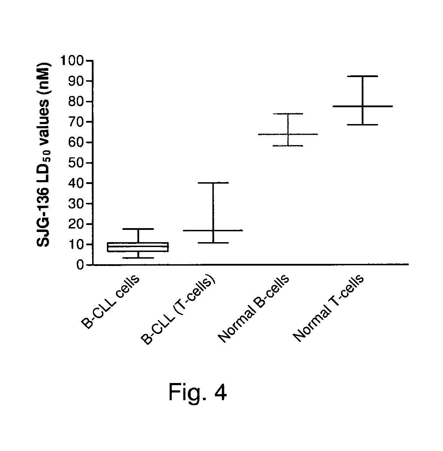

Figure 4 shows a comparison of the cytotoxicity of the compound of formula I (SJG-136) in B-CLL cells and normal lymphocytes. Cytotoxicity was compared using LD₅₀ values (± SD) derived from in vitro cultures of malignant B-cells and T-cells derived from B-CLL samples and the B- and T-lymphocyte sub-sets from normal age-matched control samples.

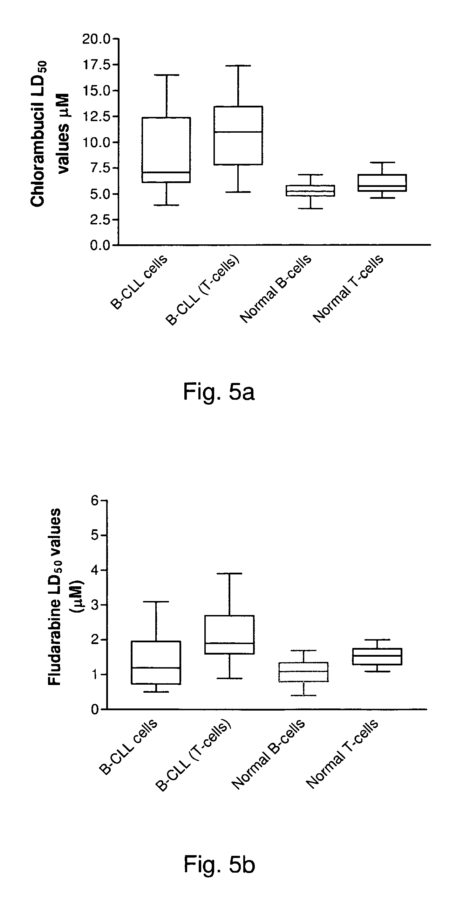

Figures 5a and 5b show comparisons of the cytotoxicity of chlorambucil and fludarabine respectively in B-CLL cells and normal lymphocytes. Cytotoxicity was compared using LD₅₀ values (± SD) derived from in vitro cultures of malignant B-cells and T-cells derived from B-CLL samples and the B- and T-lymphocyte sub-sets from normal age-matched control samples.

### Methods of Treatment

As described above, the present invention relates to a compound of formula I, or a pharmaceutically acceptable salt or solvate thereof for use,in a method of therapy of B-CLL. It is preferred that the compound of formula I is administered in the form of a pharmaceutical composition.

The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of medical doctors.

A compound may be administered alone or in combination with other treatments, either simultaneously or sequentially dependent upon the condition to be treated. Examples of treatments and therapies include, but are not limited to, chemotherapy (the administration of active agents, including, e.g. drugs); surgery; and radiation therapy.

Pharmaceutical compositions according to the present invention, and for use in accordance with the present invention, may comprise, in addition to the active ingredient, i.e. a compound of formula I, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

In pharmaceutical compositions of the present invention which comprise a compound of formula I and a solvent, the compound of formula I may preferably be present in its carbinolamine or carbinolamine ether form.

### Salts and Solvates

It may be convenient or desirable to prepare, purify, and/or handle a corresponding salt of the active compound, for example, a pharmaceutically-acceptable salt. Examples of pharmaceutically acceptable salts are discussed in Berge, et al., J. Pharm. Sci., 66, 1-19 (1977).

For example, if the compound is anionic, or has a functional group which may be anionic (e.g. -COOH may be -COO⁻), then a salt may be formed with a suitable cation. Examples of suitable inorganic cations include, alkali metal ions such as Na⁺ and K⁺, alkaline earth cations such as Ca²⁺ and Mg²⁺, and other cations such as Al⁺³. Examples of suitable organic cations include, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g. -NH₂ may be -NH₃⁺), then a salt may be formed with a suitable anion. Examples of suitable inorganic anions include, those derived from the following inorganic acids: hydrochloric, hydrobromic, hydroiodic, sulfuric, sulfurous, nitric, nitrous, phosphoric, and phosphorous.

Examples of suitable organic anions include, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucoheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, those derived from the following polymeric acids: tannic acid, carboxymethyl cellulose.

It may be convenient or desirable to prepare, purify, and/or handle a corresponding solvate of the active compound. The term "solvate" is used herein in the conventional sense to refer to a complex of solute (e.g. active compound, salt of active compound) and solvent. If the solvent is water, the solvate may be conveniently referred to as a hydrate, for example, a mono-hydrate, a di-hydrate, a trihydrate, etc.

Solvates of particular relevance to the present invention are those where the solvent adds across the imine bond of the PBD moiety, which is illustrated below where the solvent is water or an alcohol (R^{B}OH, where R^{B} is an ether substituent as described above e.g. MeOH): 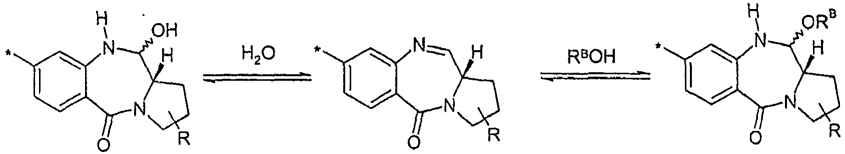 wherein * represents the dimer link to the other PBD moiety. These forms can be called the carbinolamine and carbinolamine ether forms of the PBD. The balance of these equilibria depend on the conditions in which the compounds are found, as well as the nature of the moiety itself.

In general any nucleophilic solvent is capable of forming such solvates as illustrated above for hydoxylic solvents. Other nucleophilic solvents include thiols and amines.

These solvates may be isolated in solid form, for example, by lyophilisation.

The compound of formula I may be in a solvate form, for example with water or an alcohol, such as methanol, added across the imine bond:

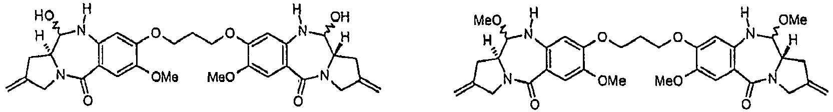

### Examples

### Example 1 : Synthesis of the PBD Dimer SJG-136

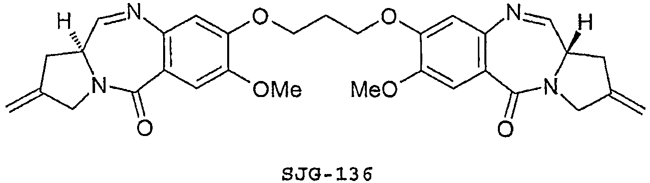

SJG-136 is a pyrrolobenzodiazepine (PBD) dimer according to formula I that is a sequence-selective DNA interstrand cross-linking agent. It comprises two PBD monomeric units ^{3,4} joined through their C8-positions via a propyldioxy linker, with each PBD C-ring containing a C2-exo-methylene functionality.^{5,6} The molecule has been shown to interact in the minor groove of DNA, spanning a total of six base pairs and alkylating the N2-positions of guanine bases situated on opposite strands of the DNA but separated by two base pairs. NMR, molecular modeling and gel electrophoresis-based studies on SJG-136 and related analogues suggest that it prefers to bind to Pu-GATC-Py sequences (Pu = purine; Py = pyrimidine), a feature that can be explained by hydrogen bonding interactions between the drug and certain molecular features of the DNA bases.⁷⁻⁹ The SJG-136 adduct provides a high degree of stabilisation towards melting of the duplex DNA as evidenced by energy calculations and an observed 33.6°C increase in the thermal denaturation of calf thymus DNA after incubation for 18 hours at 37°C with SJG-136.^{10,11} An NCI COMPARE analysis has shown that, although SJG-136 compares in general terms with DNA-binding agents, it does not fit within any of the clusters of known agents, including anthramycin and bizelesin.^{12,13}

Two methods of synthesising SJG-136 are described in published PCT application WO/0012508.

### Protocol: Induction of apoptosis in B-CLL cells

### Patient cells and clinical details

Peripheral blood samples from 34 patients with B-CLL (20 untreated and 14 treated) and 10 age-matched normal controls were obtained with the patients' informed consent. B-CLL was defined by clinical criteria as well as cellular morphology and the co-expression of CD19 and CD5 in lymphocytes simultaneously displaying restriction of light-chain rearrangement. Staging was based on the Binet classification system.¹⁴ None of the previously treated patients had received therapy for at least three months prior to this study. V_{H} gene mutational status was determined for all 34 patients using the method described previously.¹⁵ The resulting PCR products were sequenced and were considered unmutated if they showed homology of 98% or higher with the closest germ line sequence. The clinical characteristics of the patient cohort are summarized in Table 1.

**Table 1**

| | |
|---|---|
| No. of Cases | 34 |
| Mean age (years) | 67 |
| Sex (Male/Female) | 21/13 |
| Binet Stage (A/B/C) | 17/4/13 |
| Previous treatment (Untreated/Treated) | 20/14 |
| V_{H} mutation (Mutated/Unmutated) | 18/16 |

### Primary B-CLL cell culture conditions

Freshly isolated peripheral blood lymphocytes (1x10⁶ /ml) were cultured in Eagles medium (Invitrogen, Paisley, UK) supplemented with 100 units/ml penicillin, 100 *µ*g/ml streptomycin and 10% fetal calf serum. Lymphocytes were incubated at 37°C in a humidified 5% carbon dioxide atmosphere in the presence of SJG-136 (10⁻¹⁰-10⁻⁷ M). Parallel experiments using chlorambucil (10⁻⁶-5x10⁻⁵ M) : 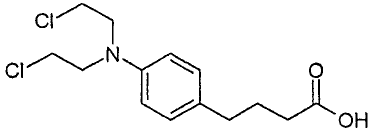 and fludarabine (10⁻⁷-10⁻⁵ M) : 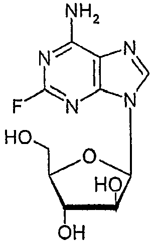 were also performed in order to assess the comparative intra- and inter-sample in vitro cytotoxicity. In addition, control cultures were carried out to which no drug was added to normal and leukemic lymphocytes. Cells were subsequently harvested by centrifugation and were analyzed by flow cytometry using the methods outlined below. Experiments were performed either in duplicate or triplicate.

### Cell lines

The ability of SJG-136 to induce apoptotic cell death was investigated in the p53 non-expressing/mutant leukemic cell lines, K562 (chronic myelogenous leukemia) and MOLT-4 (T-cell acute lymphoblastic leukemia) containing a G>A mutation at codon 248 of the p53 gene. Cells were maintained in RPMI 1640 (Invitrogen) with 10% fetal calf serum, 100 units/ml penicillin, and 100 *µ*g/ml streptomycin in a humidified atmosphere with 5% CO₂. Cells were cultured for 48h in the presence or absence of SJG-136, chlorambucil or fludarabine at the concentrations given previously. Apoptosis was measured by Annexin V labeling (Dako, Ely, UK) and was quantified using flow cytometry.

### Measurement of in vitro apoptosis

In this study changes in forward light scatter (FSC) and side light scatter (SSC) characteristics were used to quantify apoptotic and viable cell populations as described previously.¹⁶⁻¹⁸ Typically, lymphocytes show a reduction in FSC (a function of cytoplasmic shrinkage) and an increase in SSC (due to increased granularity) when they undergo apoptosis.¹⁹ The quantitation of apoptosis using an FSC/SSC gating strategy in conjunction with back gating of R-phycoerythrin cyanine 5 (RPE-cy5) labeled CD19⁺ (Dako) or R-phycoerythrin labeled CD3⁺ (Dako) lymphocytes allowed simultaneous acquisition of data in viable and apoptotic B-lymphocyte and T-lymphocyte sub-populations, respectively. All LD₅₀ and LD₉₀ values (the concentration of the relevant chemotheraputic agent required to kill 50% and 90% of cells respectively) were derived from the dose-response curves. Duplicate samples were assessed using fluorescein (FITC)-labeled Annexin V to confirm the presence of apoptotic cells in the cell cultures and to validate the FSC/SSC quantitation method.²⁰

### SJG-136 induced caspase-3 activation

B-CLL cells were incubated at 37°C in a humidified 5% carbon dioxide atmosphere in the presence of SJG-136 (10-¹⁰-10⁻⁷ M) or fludarabine (10⁻⁷-10⁻⁵ M) for 12, 24 and 48h. Cells were then harvested by centrifugation and labeled with CD19 RPE-cy5 conjugated antibody. Subsequently the cells were incubated for 1h at 37°C in the presence of the PhiPhiLux™ G₁D₂ substrate (Calbiochem, Nottingham, UK). The substrate contains two fluorophores separated by a quenching linker sequence that is cleaved by active caspase-3. Once cleaved the resulting products fluoresce green and can be quantified using flow cytometry. In additional experiments the caspase-8 inhibitor, Z-IETD-FMK, or the caspase-9 inhibitor, Z-LEHD-FMK, (Cambridge Bioscience, Cambridge, UK) were added to SJG-136-treated cell cultures (final concentration 2 µM) in order to determine whether either of these inhibitors was able to abrogate the apoptotic effects of SJG-136 in B-CLL cells. The activation of caspase-3 was partially abrogated by the addition of the caspase-9 inhibitor, Z-LEHD-FMK, but not by the caspase-8 inhibitor, Z-IETD.FMK, indicating that SJG-136-induced apoptosis is predominantly mediated through the intrinsic apoptotic pathway.

### Statistical analysis

The data obtained in these experiments were evaluated using the equal variance and paired Student's t-test, and correlation coefficients were calculated from least squares linear regression plots. LD₅₀ values were calculated from line of best-fit analysis of the dose response curves. All statistical analyses were performed using Graphpad Prism 3.0 software (Graphpad Software Inc., San Diego, CA).

### Measurement of apoptosis in B-CLL cells

A flow cytometry-based *in vitro* apoptosis detection assay was used to determine whether SJG-136 could induce apoptotic cell death in B-CLL cells. The characteristic changes in the forward and side light scatter resulting from cellular shrinkage described previously were used to define apoptosis.¹⁹ In addition, Annexin V labeling was also performed in order to verify the light scatter data. Apoptosis was induced in all 34 patient samples following exposure to SJG-136 with a mean LD₅₀ value (±SD) of 9.06 nM (± 3.2 nM) and a mean LD₉₀ value (±SD) of 43.09 nM (± 26.1 nM) (Fig. 2a and 2b respectively). There was no significant difference in the LD₅₀ values between the treated and untreated patient groups (Fig. 3a). Similarly, there was no significant difference in LD₅₀ values when the patient cohort was analyzed according to mutational status (Fig. 3b). Furthermore, two of the patients in the treated patient group had a known p53 mutation and showed a high degree of *in vitro* resistance to fludarabine but demonstrated similar sensitivity to SJG-136 when compared with the remaining patient samples.

### Example 2: Differential cytotoxicity of SJG-136 in B- and T-cells from normal and from B-CLL patients.

B- and T-lymphocytes from 10 healthy normal control patients were assessed for their sensitivity to SJG-136-induced apoptosis. In addition, the T-lymphocytes from 12 B-CLL patients from the untreated patient group, described in the protocol above, whose T-lymphocyte population was greater than 5% of the total lymphocyte population were also analyzed in order to determine whether SJG-136 had differential cytotoxic effects on the various lymphocyte subpopulations.

The T-cells from the B-CLL samples showed consistently higher LD₅₀ values than their corresponding malignant B-cell clones (P = 0.0006; paired t-test). In addition, the healthy normal control B-and T-lymphocytes demonstrated higher LD₅₀ values than the B-CLL cells (P < 0.0001 and P < 0.0001 respectively). The relative sensitivities of the various lymphocyte populations to SJG-136 are illustrated in Fig. 4.

Comparative example 1: Differential cytotoxicity of fludarabine and chloambucil in B- and T-cells from normal and from B-CLL patients. The sensitivity of normal B- and T- cells and B- and T- cells from B-CLL patients were assessed for their sensitivity to current chemotherapeutic agents fludarabine and chlorambucil using the same methods as in example 2. The LD₅₀ values for chlorambucil and fludarabine are shown in figs 5a and 5b respectively.

From figs 5a and 5b, it is clear that the mean LD₅₀ values for both chlorambucil and fludarabine are lower in normal B- and T- cells than in corresponding B- and T- cells from B-CLL patients i.e. normal cells are killed more easily by these agents than are B-CLL cells.

Comparison of figs 5a and 5b with fig. 6 clearly shows the differential killing of malignant cells over normal cells by SJG-136.

### References

1. Ries, L.A.G., Miller, B.A., Hankey, B.F., Eisner, M.P., Mariotto, A., Fay, M.P., Feuer, E.J., and Edwards, B.K. SEER cancer statistics review, 1972-1991: tables and graphs., pp 94-2789, National Cancer Institute Publication, NIH Bethesda, MD, 1994.
2. Lee, J.S., Dixon, D.O., Kantarjian, H.M., Keating, M.J., and Talpaz, M. Prognosis in chronic lymphocytic leukemia: a multivariate regression analysis of 325 untreated patients. Blood. 69: 929-936, 1987.
3. Thurston, D. E. Advances in the Study of Pyrrolo[2,1-c] [1,4]benzodiazepine (PBD) Antitumour Antibiotics. Molecular Aspects of Anticancer Drug-DNA Interactions; pp 54-88, The Macmillan Press Ltd., London, UK: London, 1993.
4. Thurston, D. E. Nucleic Acid Targeting: Therapeutic Strategies for the 21st Century. Br J Cancer, 80: 65-85, 1999.
5. Gregson, S. J., Howard, P. W., Hartley, J. A., Brooks, N. A., Adams, L. J., Jenkins, T.C., Kelland, L.R., Thurston, D.E. Design, synthesis, and evaluation of a novel pyrrolobenzodiazepine DNA-interactive agent with highly efficient cross-linking ability and potent cytotoxicity. J Med Chem., 44: 737-748, 2001.
6. Gregson, S. J., Howard, P. W., Jenkins, T. C., Kelland, L. R., and Thurston, D. E. Synthesis of a novel C2/C2 '-Exo unsaturated pyrrolobenzodiazepine cross-linking agent with remarkable DNA binding affinity and cytotoxicity. J Chem Soc Chem Commun., 797-798, 1999.
7. Mountzouris, J. A., Wang, J. J., Thurston, D., and Hurley, L. H. Comparison of a DSB-120 DNA Interstrand Cross-Linked Adduct with the Corresponding Bis-Tomaymycin Adduct - an Example of a Successful Template-Directed Approach to Drug Design Based Upon the Monoalkylating Compound Tomaymycin. J Med Chem., 37: 3132-3140, 1994.
8. Jenkins, T. C., Hurley, L. H., Neidle, S., and Thurston, D. E. Structure of a Covalent DNA Minor-Groove Adduct With a Pyrrolobenzodiazepine Dimer - Evidence For Sequence-Specific Interstrand Cross-Linking. J Med Chem., 37: 4529-4537, 1994.
9. Smellie, M., Bose, D. S., Thompson, A. S., Jenkins, T. C., Hartley, J. A., and Thurston, D.E. Sequence-Selective Recognition of Duplex DNA Through Covalent Interstrand Cross-Linking: Kinetic and Molecular Modeling Studies With Pyrrolobenzodiazepine Dimers. Biochem., 42: 8232-8239, 2003.
10. Adams, L. J.; Jenkins, T. C.; Banting, L.; Thurston, D. E. Molecular Modelling of a Sequence-Specific DNA-Binding Agent Based on the Pyrrolo[2,1-c][1,4]benzodiazepines. Pharmacy & Pharmacology Communications 1999, 5, 555-560..
11. Gregson, S. J., Howard, P. W., Gullick, D. R., Hamaguchi, A., Corcoran, K. E. et al. Linker Length Modulates DNA Cross-Linking Reactivity and Potency for Ether-Linked C2-Exo-Unsaturated Pyrrolo[2,1-c][1,4]benzodiazepine (PBD) Dimers. J Med Chem., (In Press) 2003.
12. Alley, M. C., Hollingshead, M. G., Pacula-Cox, C. M., Waud, W. R., Hartley, J. A., Howard, P.W., Gregson, S.J., Thurston, D.E., and Sausville, E.A. SJG-136 (NSC 694501) A Novel Rationally Designed DNA Minor Groove Interstrand Cross-Linking Agent With Potent and Broad Spectrum Antitumour Activity. Part 2: Efficacy Evaluations. Cancer Res., (In Revision) 2003.
13. Hartley, J. A., Spanswick, V. J., Brooks, N., Clingen, P. H., McHugh, P. J., Hochhauser, D., Pedley, R.B., Kelland, L.R., Alley, M.C., Schultz, R., Hollingshead, M.G., Sausville, E.A., Gregson, S.J., Howard, P.W., and Thurston, D.E. SJG-136 (NSC 694501) A Novel Rationally Designed DNA Minor Groove Interstrand Cross-Linking Agent With Potent and Broad Spectrum Antitumour Activity. Part 1: Cellular Pharmacology, In Vitro and Initial In Vivo Antitumor Activity. Cancer Res., (In Revision) 2003.
14. Binet, J.L., Auquier, A., Dighiero, G., Chastang, C., Piguet, H., Goasguen, J., Vaugier, G., Potron, G., Colona, P., Oberling, F., Thomas, M., Tchernia, G., Jacquillat, C., and Boivin, P. A new prognostic classification of chronic lymphocytic leukemia derived from a multivariate survival analysis. Cancer, 48: 198-206, 1981.
15. Starczynski, J., Pepper, C., Pratt, G., Hooper, L., Thomas, A., Hoy, T., Milligan, D., Bentley, P., and Fegan, C. The P2X7 receptor gene polymorphism 1513A→C has no effect on clinical prognostic markers, in vitro sensitivity to fludarabine, Bcl-2 family protein expression or survival in B-cell chronic lymphocytic leukaemia. Br J Haematol., 122: 66-71, 2003.
16. Pepper, C., Thomas, A., Hoy, T., and Bentley, P. Chlorambucil resistance in B-cell chronic lymphocytic leukemia is mediated through failed Bax induction and selection of high Bcl-2-expressing subclones. Br J Haematol., 104: 581-588, 1999.
17. Pepper, C., Hooper, K., Thomas, A., Hoy, T., and Bentley, P. Bcl-2 antisense oligonucleotides enhance the cytotoxicity of Chlorambucil in B-cell chronic lymphocytic leukemia cells. Leuk Lymphoma. 42: 491-498, 2001.
18. Pepper C, Thomas A, Hoy T. et al. The vitamin D3 analog, EB1089, induces apoptosis via a p53-independent mechanism involving p38 MAP kinase activation and suppression of ERK activity in B-cell chronic lymphocytic leukemia cells in vitro. Blood. 101: 2454-2460, 2003.
19. Ferlini, C., Di Cesare, S., Rainaldi, G., Malorni, W., Samoggia, P., Biselli, R., and Fattorossi, A. Flow cytometric analysis of the early phases of apoptosis by cellular and nuclear techniques. Cytometry, 24: 106-115, 1996.
20. Vermes, I., Haanen, C., Steffens-Nakken, H., and Reutelingsperger, C. A novel assay for apoptosis: flow cytometric detection of phosphatidylserine expression on early apoptotic cells using fluorescein labeled Annexin V. J Immunol Methods. 184: 39-51, 1995.

## Claims

1. Use of a compound in the manufacture of a medicament for the treatment of B-cell chronic lymphocytic leukaemia (B-CLL), wherein it is desired to selectively kill malignant B-cells, wherein the compound has the formula I: 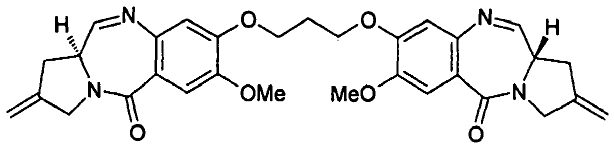 or is a pharmaceutically acceptable salt or solvate thereof.

2. A compound of formula I: 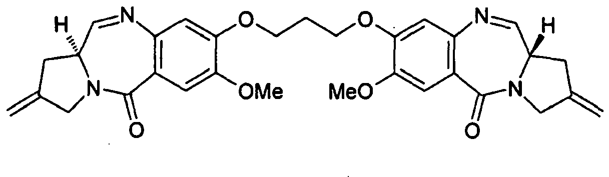 or a pharmaceutically acceptable salt or solvate thereof, for use in a method of treating B-CLL, wherein it is desired to selectively kill malignant B-cells.

3. The use according to claim 1, wherein the compound or pharmaceutically acceptable salt or solvate thereof shows a higher cytotoxicity towards malignant B-cell chronic lymphocytic leukaemia cells than towards normal B-cells.

4. A compound for use according to claim 2, wherein the compound or pharmaceutically acceptable salt or solvate thereof shows a higher cytotoxicity towards malignant B-cell chronic lymphocytic leukaemia cells than towards normal B-cells.

## Patentansprüche

1. Verwendung einer Verbindung bei der Herstellung eines Medikaments zur Behandlung von chronischer lymphozytischer B-Zell-Leukämie (B-CLL), wobei eine selektive Tötung von malignen B-Zellen erwünscht ist, wobei die Verbindung die folgende Formel I aufweist: 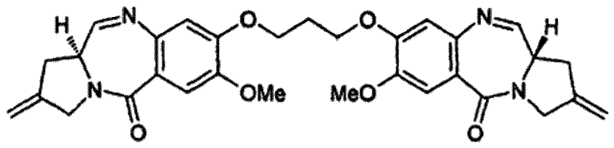 oder ein pharmazeutisch annehmbares Salz oder Solvat davon ist.

2. Verbindung der Formel I: 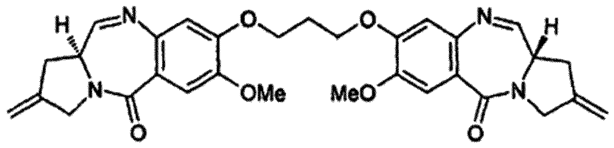 oder ein pharmazeutisch annehmbares Salz oder Solvat davon zur Verwendung in einem Verfahren zur Behandlung von B-CLL, wobei eine selektive Tötung von malignen B-Zellen erwünscht ist.

3. Verwendung nach Anspruch 1, wobei die Verbindung oder das pharmazeutisch annehmbare Salz oder Solvat davon höhere Zytotoxizität gegenüber malignen Zellen bei chronischer lymphozytischer B-Zell-Leukämie aufweist als gegenüber normalen B-Zellen.

4. Verbindung zur Verwendung nach Anspruch 2, wobei die Verbindung oder das pharmazeutisch annehmbar Salz oder Solvat davon höhere Zytotoxizität gegenüber malignen Zellen bei chronischer lymphozytischer B-Zell-Leukämie aufweist als gegenüber normalen B-Zellen.

## Revendications

1. Utilisation d'un composé dans la fabrication d'un médicament pour le traitement d'une leucémie lymphocytaire chronique à cellules B (B-CLL), dans laquelle on souhaite tuer sélectivement les cellules B malignes, dans laquelle le composé est de formule I : 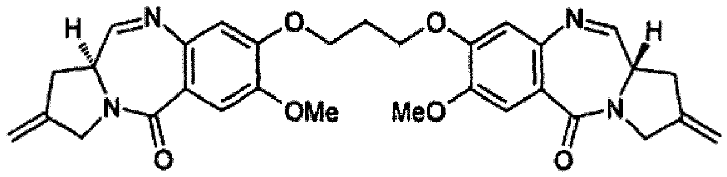 ou est un sel pharmaceutiquement acceptable ou un solvate d'un tel composé.

2. Composé de formule I : 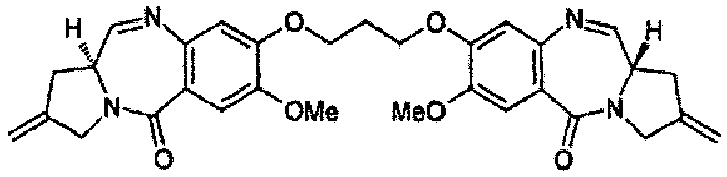 ou un sel pharmaceutiquement acceptable ou un solvate d'un tel composé, pour utilisation dans un procédé pour traiter une B-CLL, dans lequel on souhaite tuer sélectivement les cellules B malignes.

3. Utilisation selon la revendication 1, dans laquelle le composé ou son sel pharmaceutiquement acceptable ou son solvate présente une cytotoxicité vis-à-vis des cellules de leucémie lymphocytaire chronique à cellules B malignes supérieure à celle vis-à-vis des cellules B normales.

4. Composé pour utilisation selon la revendication 2, lequel composé ou son sel pharmaceutiquement acceptable ou son solvate présente une cytotoxicité vis-à-vis des cellules de leucémie lymphocytaire chronique à cellules B malignes supérieure à celle vis-à-vis des cellules B normales.
